# EUROPEAN PATENT SPECIFICATION

(11) **EP 2 696 742 B1**
(45) Date of publication and mention of the grant of the patent: **23.09.2020**
(21) Application number: 12771812.0
(22) Date of filing: 06.04.2012
(51) Int. Cl.: A61B 1/04

(54) **LASER VIDEO ENDOSCOPE**
LASERVIDEO-ENDOSKOP
ENDOSCOPE VIDÉO LASER

(30) Priority: 12.04.2011 US 201113084789
(43) Date of publication of application: 19.02.2014
(73) Proprietor: Beaver-Visitec International, Inc., Waltham, MA 02452 (US)
(72) Inventor: URAM, Martin, New Jersey, 07739 (US)
(74) Representative: Boult Wade Tennant LLP
(86) International application number: PCT/US2012/032483
(87) International publication number: WO 2012/141980

(56) References cited:
- EP-A1- 0 512 592
- DE-A1- 19 542 955
- US-A1- 2005 033 309
- US-A1- 2007 293 727
- US-B1- 6 193 650
- US-B2- 7 522 797

## Description

### Background Of The Invention

This invention relates in general to a laser video endoscope for use in ophthalmology operations and more particularly to one in which the operating probe has a small diameter so that, for example, it can be passed through a 23 gauge (0.64 mm) sleeve such as a trocar sleeve.

Laser video endoscopes are known and examples are described in Applicant's issued Patent No. 5,121,740 issued on June 16, 1992 and Patent No. 6,997,868 issued on February 14, 2006. These endoscopes used in ophthalmology operations are either disposable or reused after autoclaving or sterilization. Reuse is important because of the expense of the endoscope. These prior art endoscopes are employed with the probe passing through a 20 gauge (0.89 mm) tissue incision during ophthalmological surgery. A 20 gauge (0.89 mm) incision has been a standard in the art and is used for entry by instruments employed during an ophthalmological surgical routine.

However, a smaller 23 gauge (0.64 mm) sleeve has been employed more recently. This sleeve, such as a trocar sleeve is a tube implanted in a body wall which permits insertion and removal of a surgical instrument without touching the body wall tissue. The value of the 23 gauge (0.64 mm) sleeve is that it involves a smaller incision and therefore quicker recovery time. The 23 gauge (0.64 mm) sleeve provides an opening smaller than the 20 gauge (0.89 mm) incision and thus requires the probes thereof to be smaller in diameter so that they can fit through the 23 gauge (0.64 mm) sleeve. One problem is that a 23 gauge (0.64 mm) probe is so small in diameter (25 mils, 0.64 mm) that it is fragile and tends to break. This breakage problem becomes a major concern when using a laser video endoscope because of the cost of these endoscopes. These laser video endoscopes are used in glaucoma, retinal and vitrectomy operations.

Accordingly, it is a major purpose of this invention to provide a design for a laser video endoscope that will permit the probe to be designed so that it can be inserted through a 23 gauge (0.64 mm) sleeve and will maintain sufficient robustness so as to minimize the amount of breaking and provide the possibility for reuse of the instrument.

It is a further purpose of this invention to achieve this small probe in a design for an endoscope with which the surgeon is familiar and in a design that avoids significant added costs. This familiarity of use and reasonable cost will enhance the likelihood of use.

US 2005/0033309 relates to a small gauge surgical instrument assembly which has support along a section of the instrument that can be selected by the surgeon. EP 0 512 592 A relates to a surgical endoscope comprising an illumination zone, an image guide and a laser fiber.

### Brief Description

The present invention relates to a laser video endoscope for ophthalmologic surgery according to claim 1. Further aspects and preferred embodiments are defined in the dependent claims. Aspects, embodiments and examples of the present disclosure which do not fall under the scope of the appended claims do not form part of the claimed invention and are merely provided for illustrative purposes.

One embodiment of the surgical instrument of this invention employs a stainless steel probe having a distal portion and a proximal portion. The distal portion has an outer diameter (OD) that is less than 25 mils (thousandths of an inch) (0.64 mm) with a two mil (0.05 mm) wall thickness. Thus it can be inserted through a 23 gauge (0.64 mm) sleeve. The proximal portion of the probe, exiting from the hand piece, has a 31 mil (0.79 mm) OD and a wall thickness of five mils (0.1 mm). The distal 25 mils (0.64 mm) diameter portion has a length of 710 mils (18 mm). This combination of three design features provides a probe that can fit through a 25 mil (23 gauge) (0.64 mm) sleeve yet be robust enough to minimize the risk of breaking. Most breakage occurs at the juncture between the hand piece and the probe.

In addition the laser video endoscope has the known elements of a source of illumination, source of laser energy and camera assembly. All of these three elements are coupled by optical fibers through the hand piece and then through the surgical probe to provide illumination, image transmission and laser operating energy.

However the instrument of this invention provides a trade-off between the size of the optical cabling used for the three functions of illumination, imaging and delivering laser energy. A particular trade-off is required to meet the dimensional limitations of the 23 gauge (0.64 mm) probe and yet adequately provide these three functions. The trade off made by this invention between adequate functioning and dimensional limitations is one that results in a 100 micron laser fiber (0.1 mm), a 6,000 fiber image bundle having a 14 mil (0.36 mm) diameter circular configuration and an illumination bundle having 210 fibers that fills the 21 mil (0.53 mm) inner diameter of the distal portion of the probe 28.

The small diameter laser fiber requires laser energy that is well collimated, having little dispersion so that no laser energy is wasted. A so called green laser having a wavelength of 532 nanometers is used.

### Brief Description Of The Drawings

FIG. 1 is a schematic illustration of a prior art system extending from the probe 10 to the terminals 22, 24 and 26.
FIG. 2 is an illustration of an embodiment of this invention showing the cable, hand piece and probe.
FIG. 3 is a cross sectional view through the small diameter distal portion of the probe of the FIG. 2 device.
FIG. 4 shows the location of the laser filter at a position distal of the camera.

### Detailed Description

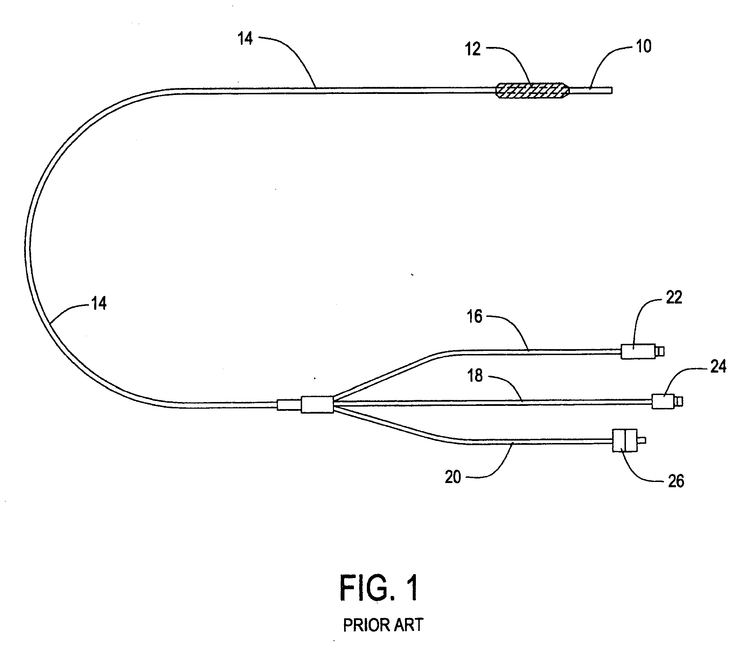

FIG. 1 illustrates one prior art device. The rest of the figures are all to a single embodiment of the device of this invention.

As shown in FIG. 1, the known video endoscope has an operating probe 10, a hand piece 12 and a cable 14. Extending through the probe, the hand piece and cable are a laser guide 16, illumination guide 18, and an image guide 20. These are all fiber optic guides which extend from the distal end of the probe 10 to the terminals 22, 24 and 26.

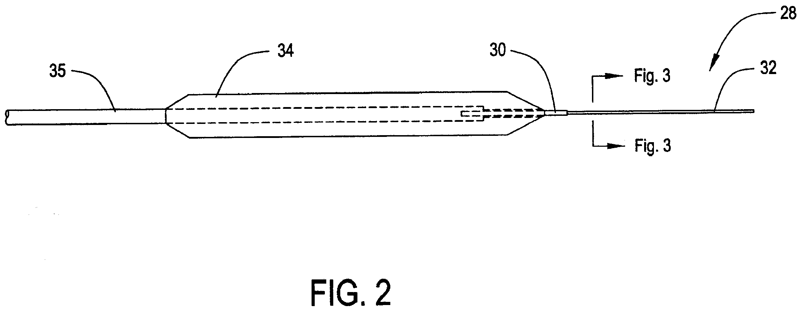

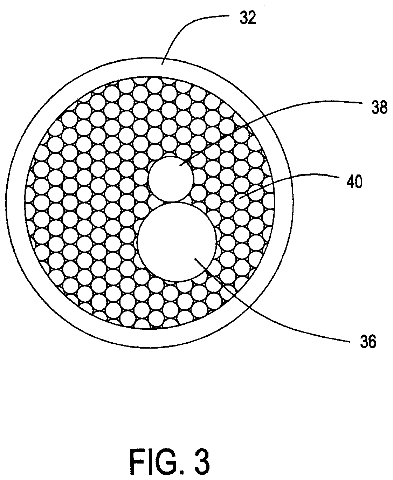

FIGs. 2 through 4 illustrate an embodiment of this invention showing probe 28, hand piece 34 and cable 35. The probe 28 has a proximal portion 30 and a distal portion 32. The proximal portion 30 has a 20 gauge (35 mil, 0.89 mm) outer diameter and a five mil (0.1 mm) wall thickness. The probe is stainless steel. The proximal portion extends into the hand piece 34. Thus, at the juncture of the end of the hand piece 34 and the probe 28, there is a diameter having sufficient robustness to contribute to minimizing the likelihood of breaking at the juncture between the hand piece and the probe.

The length of the proximal portion 30 of the probe is 120 mils (3 mm) and the length of the distal portion 32 is 710 mils (18 mm) for a probe length of 830 mils (21 mm). The distal portion 32 of the probe 28 has an outer diameter of 25 mils (0.64 mm) and will be able to extend through a 23 gauge (0.64 mm) sleeve to provide illumination and laser energy delivery within the eye during a surgical procedure and to transmit image from the eye. This distal portion 32 has a wall thickness of two mils (0.05 mm) and a length of 710 mils (18 mm). The 710 mil (18 mm) length is long enough for most applications and short enough to minimize breaking. It has been found that this short a length for the distal portion 32 contributes to the robustness of the probe 28. These dimensional values can be varied slightly to provide a probe that can be used with other small size sleeves.

This 25 mil (0.64 mm) diameter probe has to meet the need of providing enough light and enough laser energy while maintaining an adequate image guide. In order to obtain a useable viable surgical instrument that provides adequate illumination energy, imaging and laser energy, trade-off s are made of these various light fiber functions that will provide something useable by the surgeon. What Applicant has done is to provide a particular tradeoff of dimensions for each of these light fibers.

Essentially, the trade-off involves a standard minimum size image guide 36, a very much reduced laser guide 38 having a 100 micron (0.1 mm) diameter instead of a 200 micron (0.2 mm) diameter and a illumination light bundle 40 having only 210 fibers. This is all contained within the distal portion 32 of the probe 28 having an outside diameter of approximately 25 mils (0.64 mm), a two mil (0.05 mm) wall thickness and an inner diameter of 21 mils (0.53 mm).

This small diameter probe 28 is fragile and risks breaking off at the juncture of the hand piece 34. It has been found that the probe will be robust enough to minimize breakage by a combination of (a) a rigid, preferably metallic, probe 28, (b) a probe 28 having the two diameter design at 30 and 32 and (c) a distal segment 32 limited in length to no more than about 710 mils (18 mm). Thus the embodiment shown and tested has the following three features. The proximal portion 30 of the probe 28 has a 35 mil (0.89 mm) outside diameter that extends through the hand piece 34 and that has at least a five mil (0.1 mm) wall thickness. The distal portion of the probe 28 has a 25 mil (0.64 mm) outer diameter with a two mil (0.05 mm) wall thickness.

It has been found that such a design provides sufficient illumination to illuminate a 90 degree field. One of the compromises made in order to get a small diameter probe was to reduce the laser guide 38 fiber diameter from 200 microns (0.2 mm) to 100 microns (0.1 mm). It became important, as part of the tradeoffs involved herein, to use a 532 nanometer (nm) laser which is also known as a green laser. This 532 nm laser is more coherent and less divergent than the wavelengths now currently used such as the 810 nm laser. Accordingly, the use of this 532 nm laser in combination with the reduced size of the laser guide fiber 38 provides a reasonable amount of laser energy for the ophthalmological operations involved. This ultimately makes possible the small diameter probe.

The image guide 36 is 6,000 fibers. It is a standard 14 mil (0.36 mm) diameter off the shelf imaging bundle having adequate resolution of the image for use by the surgeon. A gradient index lens having a 14 mil (0.36 mm) diameter could be used instead of the fiber optic bundle.

However, the illumination light bundle 40 is reduced from approximately 220 fibers to about 70 fibers thereby materially contributing to the smaller diameter probe.

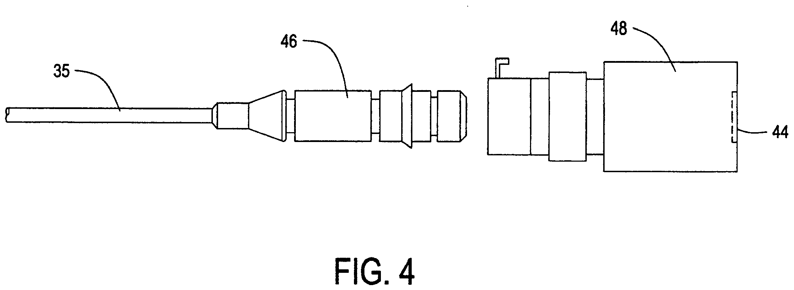

As shown in FIG. 4, the video connector 46 is coupled through known focus mechanism 48 to a camera. The laser filter 44 is mounted on a lens inside the focus mechanism 48. The laser filter 44 is used to block the laser energy from impinging on the image presented to the surgeon. The transparency of the filter is important because this laser wave length is visible and the duration of these 532 nm laser flashes can be fairly long. The pulse length can be selected as desired by the surgeon to provide the required tissue ablation.

This invention has been described in connection with an embodiment that permits use with a 23 gauge (0.64 mm) sleeve. It should be understood that variations could be made to adapt the design described to use with sleeves having variations on the 23 gauge (0.64 mm) or to be used without a sleeve. This invention is in the combination of a number of features and tradeoffs designed to work together to provide an operable and useful laser video endoscope having a small probe that provides access for eye operations with minimum trauma and reduced healing time.

## Claims

1. A laser video endoscope for ophthalmologic surgery comprising a hand piece (34) and a hollow rigid probe (28) extending distally of the hand piece that can be adapted to pass through a 23 gauge (0.64 mm) sleeve, the probe comprising:
a distal portion (32), and
a proximal portion (30), the proximal portion (30) extending into the hand piece (34),
wherein:
said distal portion of said probe having approximately a 25 mil (0.64 mm) outer diameter, approximately a 2 mil (0.05mm) thick sidewall and approximately a 710 mil (18 mm) length,
said proximal portion of said probe having at least an approximately 35 mil (0.89mm) outer diameter and at least an approximately five mil (0.1 mm) thick sidewall,
and wherein the laser video endoscope further comprises:
a laser guide fiber (38) wherein the laser guide fiber (38) is approximately 100 microns (0.1 mm) in diameter,
an imaging component (36) wherein the imaging component (36) is approximately 14 mils (0.36mm) in diameter, and
an illumination fiber bundle (40) wherein said illumination bundle comprises approximately 210 fibers, and wherein
within said approximately 2 mil (0.05mm) thick sidewall of said distal portion (32):
the laser guide fiber (38) has a cross sectional first surface,
the imaging component (36) has an essentially contiguous circular cross sectional second surface, and
the illumination fiber bundle (40) surrounds the laser guide fiber (38) and the imaging component (36), and
the first surface and the second surface do not overlap.

2. The endoscope of claim 1, wherein said imaging component comprises a fiber optic bundle having approximately 6,000 fibers.

3. The endoscope of claim 1, wherein said imagining component is a gradient index lens having a 14 mil (0.36 mm) diameter.

4. The endoscope of any previous claim wherein said rigid probe is metal.

5. The endoscope of any previous claim, wherein:
said laser fiber is adapted to transmit approximately 532 nanometer laser energy,
and wherein the laser video endoscope further comprises:
a camera coupled to said imaging component, and
a blocking filter positioned between said imaging component and said camera to block wavelengths of said laser energy, said filter being otherwise transparent to visible light.

## Patentansprüche

1. Laservideo-Endoskop für die ophthalmologische Chirurgie, umfassend ein Handstück (34) und eine hohle, starre Sonde (28), die sich distal zum Handstück erstreckt und die dazu ausgelegt sein kann, durch eine Hülse mit Kaliber 23 (0,64 mm) hindurchgeführt zu werden, wobei die Sonde umfasst:
einen distalen Abschnitt (32), und
einen proximalen Abschnitt (30), wobei sich der proximale Abschnitt (30) in das Handstück (34) hinein erstreckt, wobei:
der distale Abschnitt der Sonde einen Außendurchmesser von ungefähr 25 mil (0,64 mm), eine ungefähr 2 mil (0,05 mm) dicke Seitenwand und eine Länge von ungefähr 710 mil (18 mm) aufweist,
wobei der proximale Abschnitt der Sonde mindestens einen Außendurchmesser von ungefähr 35 mil (0,89 mm) und eine ungefähr fünf mil (0,1 mm) dicke Seitenwand von mindestens aufweist,
und wobei das Laservideo-Endoskop ferner umfasst:
eine Laserleitfaser (38), wobei die Laserleitfaser (38) einen Durchmesser von etwa 100 Mikrometer (0,1 mm) aufweist,
eine bildgebende Komponente (36), wobei die bildgebende Komponente (36) einen Durchmesser von etwa 0,36 mm (14 mil) aufweist, und
ein Beleuchtungsfaserbündel (40), wobei das Beleuchtungsbündel etwa 210 Fasern umfasst, und wobei
innerhalb der etwa 2 mil (0,05 mm) dicken Seitenwand des distalen Abschnitts (32):
die Laserleitfaser (38) eine erste Querschnittsfläche aufweist,
die bildgebende Komponente (36) eine im Wesentlichen zusammenhängende zweite Fläche mit kreisförmigem Querschnitt aufweist, und
das Beleuchtungsfaserbündel (40) die Laserleitfaser (38) und die Bildgebende Komponente (36) umgibt, und
die erste Fläche und die zweite Fläche nicht überlappen.

2. Endoskop nach Anspruch 1, wobei die bildgebende Komponente ein faseroptisches Bündel mit etwa 6.000 Fasern umfasst.

3. Endoskop nach Anspruch 1, wobei die bildgebende Komponente eine Gradientenindexlinse mit einem Durchmesser von 14 mil (0,36 mm) ist.

4. Endoskop nach einem vorherigen Anspruch, wobei die starre Sonde Metall ist.

5. Endoskop nach einem vorherigen Anspruch, wobei:
die Laserfaser dazu ausgelegt ist, eine Laserenergie von ungefähr 532 Nanometer zu übertragen, und wobei das Laservideo-Endoskop ferner umfasst: eine Kamera, die mit der bildgebenden Komponente gekoppelt ist, und
einen Sperrfilter, der zwischen der bildgebenden Komponente und der Kamera angeordnet ist, um Wellenlängen der Laserenergie zu sperren, wobei der Filter ansonsten für sichtbares Licht durchlässig ist.

## Revendications

1. Endoscope laser vidéo pour chirurgie ophtalmologique comprenant une pièce à main (34) et une sonde rigide creuse (28) s'étendant de manière distale par rapport à la pièce à main qui peut être adaptée pour passer à travers un manchon de calibre 23 (0,64 mm), la sonde comprenant :
une partie distale (32), et
une partie proximale (30), la partie proximale (30) s'étendant dans la pièce à main (34),
dans lequel :
ladite partie distale de ladite sonde ayant un diamètre externe d'environ 25 mil (0,64 mm), une paroi latérale d'épaisseur d'environ 2 mil (0,05 mm) et une longueur d'environ 710 mil (18 mm),
ladite partie proximale de ladite sonde ayant au moins un diamètre externe d'environ 35 mil (0,89 mm) et au moins une paroi latérale d'épaisseur d'environ cinq mil (0,1 mm),
et l'endoscope laser vidéo comprenant en outre :
une fibre de guidage laser (38) où la fibre de guidage laser (38) a un diamètre d'environ 100 microns (0,1 mm),
un composant d'imagerie (36) où le composant d'imagerie (36) a un diamètre d'environ 14 mil (0,36 mm), et
un faisceau de fibres d'éclairage (40) où ledit faisceau d'éclairage comprend environ 210 fibres, et où
dans ladite paroi latérale d'épaisseur d'environ 2 mil (0,05 mm) de ladite partie distale (32) :
la fibre de guidage laser (38) a une première surface en coupe transversale,
le composant d'imagerie (36) a une deuxième surface en coupe transversale circulaire essentiellement contiguë, et
le faisceau de fibres d'éclairage (40) entoure la fibre de guidage laser (38) et le composant d'imagerie (36), et
la première surface et la deuxième surface ne se chevauchent pas.

2. Endoscope de la revendication 1, dans lequel ledit composant d'imagerie comprend un faisceau de fibres optiques ayant environ 6000 fibres.

3. Endoscope de la revendication 1, dans lequel ledit composant d'imagerie est une lentille à gradient d'indice ayant un diamètre de 14 mil (0,36 mm).

4. Endoscope de l'une quelconque des revendications précédentes, dans lequel ladite sonde rigide est en métal.

5. Endoscope de l'une quelconque des revendications précédentes, dans lequel :
ladite fibre laser est adaptée pour transmettre une énergie laser d'environ 532 nanomètres,
et l'endoscope laser vidéo comprenant en outre :
une caméra couplée audit composant d'imagerie, et
un filtre de blocage positionné entre ledit composant d'imagerie et ladite caméra pour bloquer les longueurs d'onde de ladite énergie laser, ledit filtre étant par ailleurs transparent à la lumière visible.
